# EUROPEAN PATENT SPECIFICATION

(11) **EP 3 590 353 B1**
(45) Date of publication and mention of the grant of the patent: **29.10.2025**
(21) Application number: 18760923.5
(22) Date of filing: 28.02.2018
(51) Int. Cl.: A23K 10/16, A23K 10/14, A23K 10/30, A23L 11/30, A23K 10/12, C12R 1/07, C12R 1/125

(54) **METHOD FOR MANUFACTURING FERMENTED GUAR MEAL**
VERFAHREN ZUR HERSTELLUNG VON FERMENTIERTEM GUARKERNMEHL
PROCÉDÉ DE FABRICATION D'UNE FARINE DE GUAR FERMENTÉE

(30) Priority: 28.02.2017 KR 20170026703
(43) Date of publication of application: 08.01.2020
(73) Proprietor: CJ Cheiljedang Corporation, Seoul 04560 (KR)
(72) Inventor: KANG, Kyeongil, Incheon 22741 (KR); CHO, Seong Jun, Seoul 04971 (KR); RYU, Jehoon, Seoul 06990 (KR); CHANG, Kyung Hoon, Yongin-si Gyeonggi-do 16833 (KR); PARK, Seung Won, Yongin-si Gyeonggi-do 16894 (KR)
(74) Representative: SONN Patentanwälte GmbH & Co KG
(86) International application number: PCT/KR2018/002457
(87) International publication number: WO 2018/160000

(56) References cited:
- CN-A- 102 334 601
- CN-A- 105 010 758
- CN-A- 105 410 364
- CN-A- 105 795 099
- KR-A- 20110 027 535
- KR-A- 20150 129 238
- KR-A- 20160 022 423
- KR-A- 20160 084 607
- KR-B1- 101 517 326
- DATABASE BIOSIS [online] BIOSCIENCES INFORMATION SERVICE, PHILADELPHIA, PA, US; 2017, JANNATHULLA R ET AL: "Effect of fungal fermentation on the nutrient digestibility of guar meal in Penaeus vannamei", XP002799541, Database accession no. PREV201800043825
- INDIAN JOURNAL OF FISHERIES, vol. 64, no. 3, 2017, pages 67 - 74, ISSN: 0970-6011, DOI: 10.21077/IJF.2017.64.3.68434-10
- IMTIAZ, S.: "Production of alkaline protease by Bacillus subtilis using solid state fermentation", AFRICAN JOURNAL OF MICROBIOLOGY RESEARCH, vol. 7, no. 16, 16 April 2013 (2013-04-16), pages 1558 - 1568, XP055544366, Retrieved from the Internet <URL:DOI:10.5897/AJMR12.1845>

## Description

### Technical Field

The present disclosure relates to a method of preparing a fermented guar meal, a fermented guar meal prepared by the method, and a feed composition including the fermented guar meal. The invention is defined by the appended claims.

### Background Art

Guar (*Cyamopsis tetragonoloba*) belongs to the family *fabaceae,* and is used as an alternative to locust bean gum.

Guar seed is known to be composed of 14% to 18% hull, 34% to 42% endosperm, and 43% to 47% germ. In particular, guar endosperm includes a large amount of galactomannan gum (guar gum), and it is known that guar endosperm is used as a food additive such as an emulsifier, a thickener, and a food adhesive as well as used in fracking (hydraulic fracturing) extraction of shale oil and gas.

Guar germ and guar hull are generated as by-products during guar gum production. Guar meal refers to a mixture of the germ and hull, and is generally used as a raw material for feeds. However, guar meal has a relatively low protein content, as compared with animal proteins, and is poor in the contents of essential amino acids, vitamins, minerals, and UGF (Unknown Growth Factor), as compared with animal proteins in feeds for young livestock, such as fish meal, milk, etc., and includes anti-nutritional factors (ANFs) such as trypsin inhibitors, saponins, residual guar gum, and tannins. For this reason, when guar meal is used as feeds, it may reduce digestibility of livestock. In particular, since anti-nutritional factors such as trypsin inhibitors (TI) and saponins may reduce digestibility of young livestock and may inhibit growth thereof, it is not added to feeds for young livestock and its use in feeds for growing pigs or cattle is also very limited.

In order to solve these problems of guar meal, concentrated guar proteins have been developed, but there is a disadvantage in that its production cost is high. Further, a guar meal fermentation method of using complex strains and an anaerobic solid-state fermentation method of using lactic acid bacteria and yeasts have been suggested (Chinese Patent Publication Nos. 2015-10384290 and 2010-10228079). CN 105 795 099 A discloses a method for preparing a fermented guar meal prepared by mixed microbial solid fermentation. However, these methods have various problems in that a long fermentation time and a drying process are required and quality control is also required due to use of anaerobic complex strains and a continuous conveyor.

### Disclosure

### Technical Problem

Under this background, the present inventors have made intensive efforts to solve the above problems, and as a result, they found that when a guar meal is fermented with a *Bacillus* strain, nutritional components and digestibility may be improved, thereby completing the present disclosure.

### Technical Solution

An object of the present disclosure is to provide a method of preparing a fermented guar meal, the method including the steps of (1) pretreating a guar meal; (2) seeding a *Bacillus* strain in the pretreated guar meal; and (3) solid-state fermenting the strain seeded in the guar meal to obtain the fermented guar meal. The method of preparing a fermented guar meal according to the present invention comprises the following steps of:
(1) pretreating a guar meal;
(2) seeding a Bacillus strain in an amount of 5% by weight to 20% by weight based on the weight of the guar meal in the pretreated guar meal;
(3) solid-state fermenting the strain seeded in the guar meal to obtain the fermented guar meal;
and (4) drying and pulverizing the fermented guar meal;
wherein the pretreatment step of the step (1) includes the steps of (1-1) adding water to the guar meal to control a water content thereof; (1-2) heat-treating the water content-controlled guar meal; and (1-3) cooling the heat-treated guar meal,
wherein the water content-controlled guar meal of step (1-1) has a water content of 30% to 60%(w/w),
wherein the heat treatment of the step (1-2) is performed at 85°C to 100°C for 20 minutes to 35 minutes,
wherein the cooling of the step (1-3) is performed at a temperature of 20°C to 60°C,
and wherein the Bacillus strain is Bacillus amyloliquefaciens.

Another object of the present disclosure is to provide a fermented guar meal prepared by the above method.

Still another object of the present disclosure is to provide a feed composition including the fermented guar meal.

### Advantageous Effects

A method of preparing a fermented guar meal of the present disclosure may be used to prepare a fermented guar meal which is a high-quality plant protein source having improved nutritional components and digestibility, thereby providing a protein feed composition including the fermented guar meal, the protein feed composition having a reduced feed blending ratio and probiotics effects. In addition, viscosity of guar gum in the guar meal may be reduced, and in particular, contents of indigestible oligosaccharides and trypsin inhibitors (TI) in the guar meal may be remarkably reduced, thereby preparing a high-quality fermented guar meal having more improved digestibility.

### Description of Drawings

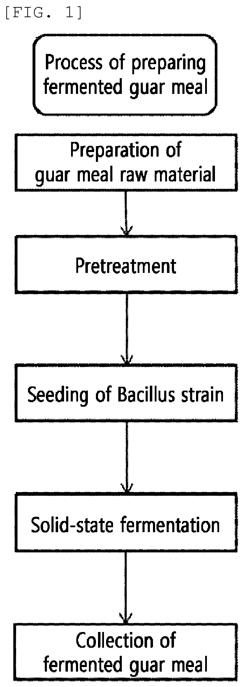

FIG. 1 is a flow chart illustrating each step of a method of preparing a fermented guar meal of the present disclosure;

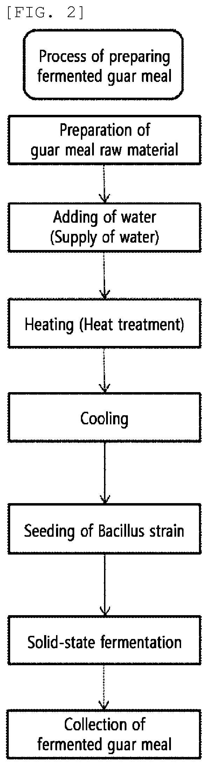

FIG. 2 is a flow chart specifically illustrating a pretreatment step in the method of preparing the fermented guar meal of the present disclosure;
FIG. 3 is a graph showing changes in viscosity of guar gum according to the type and concentration of enzymes; and
FIG. 4 is a graph showing degree of hydrolysis of guar meal proteins according to the kind of strains.

### Best Mode

In one aspect to achieve the above objects, the present disclosure provides a method of preparing a fermented guar meal according to claims 1-6.

Guar (*Cyamopsis tetragonoloba*), also called cluster bean, is a plant belonging to the family *fabaceae,* and grows up to 2 m to 3 m and lives in symbiosis with nitrogen-fixing bacteria. Mature guar is mainly used as a feed for livestock, and also used as a green manure to provide nutritional components for soils. Beans of guar contain many nutrients, but it is not easy to use guar beans for food, unless anti-nutritional factors such as trypsin inhibitors are removed therefrom.

Guar gum, also called guaran, refers to galactomannan contained in guar. Guar gum is a polysaccharide consisting of galactose and mannose, and is a substance widely used in chemical and medical fields.

Guar meal is a by-product generated during production of guar gum, and is a mixture of about 25% germs and about 75% hulls. Although guar meal may be used as a feed composition due to abundant proteins, there is a problem of low digestibility due to anti-nutritional factors, etc.

In the present disclosure, to solve the problems that are generated when guar meal is used as a feed composition, it was demonstrated that when the guar meal is fermented with a *Bacillus* amyloliquefaciens strain, the content of crude protein may be increased, indigestible oligosaccharides may be decreased, and anti-nutritional factors such as trypsin inhibitors may be reduced to increase digestibility, and therefore, a fermented guar meal fermented with the *Bacillus* amyloliquefaciens strain may be used as a feed composition.

The step of pretreating the guar meal of the step (1) includes the steps of (1-1) adding water to the guar meal tc control a water content thereof; (1-2) heat-treating the water content-controlled guar meal; and (1-3) cooling the heat-treated guar meal.

The step (1-1) is a water-adding step of adding water to the guar meal to control the water content thereof, and with respect to the purpose of the present disclosure, the step corresponds to a pretreatment step which is necessary for fermentation of the guar meal by seeding the bacteria in the guar meal. As a method of adding water to the guar meal, a method known in the art may be used without limitation.

The water content-controlled guar meal of the step (1-1) has a water content of 30% to 60%(w/w), specifically 35% to 50%(w/w), and more specifically 40% to 45%(w/w). The guar meal having a water content within the above range may prevent delay of the fermentation due to low moisture, may improve a problem of requiring high costs in a drying process after transferring and fermenting the guar meal, and may be advantageous in terms of heat efficiency.

When the water content is too low, sufficient fermentation of the guar meal does not occur, thereby generating a problem of quality deterioration due to contaminants. When the water content is too high, for example, the water content is 50% or more, there is no problem in the quality, but it is difficult to design the process, and workability and production cost competitiveness may be significantly deteriorated in the process of solid-state fermentation.

The step (1-2) is a heating step of heat-treating the water content-controlled guar meal, and the purpose of the heat treatment is to kill germs in the raw guar meal, to destroy cell walls of the guar meal, and to denature proteins, thereby providing an environment in which the desired microorganisms are able to grow vigorously. The heat treatment may be performed using various methods known in the art.

When the heat treatment temperature is low or when the treatment time is short, there is a problem that the bactericidal effect on the germs may be lowered and the subsequent fermentation process does not proceed favorably. When the heat treatment temperature is high or when the treatment time is long, digestibility may be reduced due to denaturation of proteins in the guar meal, and therefore, there is a problem in that quality of a final product may be deteriorated.

Through this heat treatment process, most contaminants present in the guar meal are killed, a chemical environment in which the subsequent fermentation process may proceed favorably may be produced, and it is expected to reduce anti-nutritional factors such as trypsin inhibitors (TI) which inhibit digestibility. When the heat treatment temperature is low or when the treatment time is short, there are problems in that reduction of anti-nutritional factors such as TI may not occur, and microorganisms in the guar meal raw material, e.g., heterologous *Bacillus* strains, fungi, and pathogens may not be killed.

The heat treatment of the step (1-2) is to treat the guar meal at 85°C to 100°C for 20 minutes to 35 minutes, and specifically at 90°C to 95°C for 25 minutes to 30 minutes. The guar meal may be treated at a temperature of 100°C or less without thermodynamic pressurization.

The step (1-3) is to cool the heat-treated guar meal and to seed the *Bacillus* strain in the guar meal, and is a process required to induce fermentation by cooling the temperature of the guar meal which is increased in the heating step and by seeding the *Bacillus* strain as a fermentation strain.

The cooling may be naturally performed after terminating the heat treatment. In order to prevent overheating by increasing the cooling rate and to perform uniform cooling, the cooling may be easily performed through a conveying process using a conveyor-type cooling apparatus.

The cooling of the step (1-3) is performed at a temperature of 20°C to 60°C, and more specifically at a temperature of 30°C to 50°C.

The *Bacillus* strain of the step (1-3) is *Bacillus amyloliquefaciens,* and more specifically, four kinds of *Bacillus amyloliquefaciens* (*Bacillus amyloliquefaciens* K2G (Korean Patent NO. 10-1517326), ATCC 23842, ATCC 23843, and ATCC 23845). As a method of culturing the *Bacillus* strain, a method known in the art may be used without limitation, and specifically, the method may be a liquid-state culture.

The *Bacillus* strain is seeded in an amount of 5% by weight to 20% by weight based on the weight of the guar meal.

In the present disclosure, the step (3) is a step of solid-state fermenting the strain seeded in the guar meal to obtain the fermented guar meal, and this step corresponds to culture and fermentation steps in the method of preparing the fermented guar meal of the present disclosure.

The culture may be performed in a solid state, and for example, the culture may be to perform fermentation using a packed-bed fermentor, etc.

As used herein, the term "solid-state fermentation" refers to culturing a microorganism or cells of an animal or a plant on a solid medium such as agar, gelatin, etc., and as the method, a method known in the art may be used without limitation.

The packed-bed fermentor may include various types such as a batch-type aeration fermentor, a close-type fermentor, a continuous aeration fermentor, etc., and any type may be used without particular limitations in the method of the present disclosure, as long as it is useful for solid-state fermentation of the guar meal. An appropriate apparatus may be selected according to a production scale and then used.

A general fermentation process may involve a solid fermentation process of 48 hours to 72 hours when yeasts, lactic acid bacteria, or fungi are used, because biological activities such as metabolisms and enzymatic activities may differ according to each strain. However, when the *Bacillus* strain is used, the fermentation process may be terminated within 24 hours, and specifically, may be performed for about 16 hours.

Specifically, the solid-state fermenting of the step (3) may be performed at a temperature of 30°C to 50°C for 15 hours to 25 hours, and more specifically, at a temperature of 37°C to 45°C for 14 hours to 16 hours.

In the fermented guar meal prepared in the step (3), a considerable number of live bacteria may exist, thereby exhibiting probiotics effects. The number of the live bacteria may be specifically 10⁷ CFU/g to 10⁸ CFU/g, but is not limited thereto.

As used herein, the term "probiotics" refer to live microorganisms that enter the body to exhibit beneficial effects on health.

The method of the present disclosure further includes the step (4) of drying and pulverizing the fermented guar meal after the above step (3).

During fermentation, a part of water in the guar meal is evaporated, but the content of remaining water immediately after terminating the fermentation is considerably high. However, so as to control a final water content in a fermented guar meal product, a drying process is further needed.

Further, when the solid-state fermentation is performed using the *Bacillus amyloliquefaciens* strain according to the present disclosure, the conditions of the fermented guar meal are very favorable, but it forms a loose aggregate in part. Therefore, it is necessary to pulverize the fermented guar meal to a uniform particle size after drying.

The drying and pulverizing may be performed by various methods known in the art. However, when the drying is performed at an excessively high temperature, most live bacteria in the fermented guar meal may be killed, and therefore, it requires caution. For this reason, the drying must be performed at a low temperature at which live bacteria are not killed, and may be performed using hot air with a low temperature and low humidity, but is not limited thereto. The pulverizing may be performed to pulverize the fermented guar meal in various sizes depending on the intended purpose of use. The pulverization method may be performed using, for example, a hammer mill.

In another aspect of the present disclosure, the present disclosure provides a fermented guar meal prepared by the method of preparing the fermented guar meal.

The fermented guar meal includes *Bacillus* amyloliquefaciens.

When a guar meal is fermented using the *Bacillus amyloliquefaciens* strain according to the above-described method of the present disclosure, it is possible to obtain a fermented guar meal in which various anti-nutritional factors including TI in the guar meal may be reduced, digestibility may be improved due to hydrolysis and low molecularization of proteins, and the content of crude protein may be increased. Since this fermented guar meal has an improved absolute value as a feed, it is a high-quality feed material as a substitute for animal proteins, and thus, its availability is very high.

Further, the guar meal includes 4% to 6% oligosaccharides. In the oligosaccharides, raffinose and stachyose which are indigestible oligosaccharides, *i.e.,* galacto-oligosaccharides (hereinafter, referred to as GOS) occupy 2% to 3%. The indigestible oligosaccharides are problematic in that they may be used as a nutritional source of harmful intestinal microorganisms or may cause diarrhea. However, the content of indigestible oligosaccharides in the guar meal fermented according to the method of the present disclosure is considerably reduced. Thus, when the fermented guar meal is used as a feed composition, it may exhibit excellent digestibility.

Specifically, the content of trypsin inhibitors in the fermented guar meal prepared by the method of the present disclosure may be about 30% to about 50%, based on the content of trypsin inhibitors (TI) in a guar meal which does not undergo the steps (1) to (3). Further, the content of indigestible oligosaccharides in the fermented guar meal prepared by the method of the present disclosure may be about 10% to about 40%, based on the content of indigestible oligosaccharides in a guar meal which does not undergo the steps (1) to (3).

Further, the fermented guar meal of the present disclosure has no problem in preference, and its feed conversion ratio is also similar to that of soybean meal, and therefore, the fermented guar meal may be widely used as a protein source to replace soybean meal.

In still another aspect of the present disclosure, the present disclosure provides a feed composition including the fermented guar meal prepared by the method of preparing the fermented guar meal.

As used herein, the term "feed composition" refers to a material providing organic or inorganic nutrients which are necessary to maintain life of a subject or to raise the subject. The feed composition may include nutrients such as energy, proteins, lipids, vitamins, minerals, etc. which are required by a subject which consumes the feed. The feed composition may be, but is not particularly limited to, a plant-based feed such as grains, nuts, food by-products, algae, fibers, oils, starch, meals, grain by-products, *etc*., or an animal-based feed such as proteins, inorganic substances, fats, minerals, single-cell proteins, zooplanktons, fish meals, etc. In the present disclosure, the feed composition is a concept encompassing a substance added to a feed (*i.e.,* a feed additive), a feed raw material, or a feed itself fed to a subject.

The subject refers to a subject to be raised, and may include any living organism without limitation, as long as it is able to intake the feed of the present disclosure. Accordingly, the feed composition of the present disclosure may be applied to diets, *i.e.,* feeds for a large number of animals including mammals, poultry, fish, and crustaceans. It may be used for mammals such as pigs, cattle, goats, *etc*. which are commercially important, zoo animals such as elephants, camels, *etc*., livestock such as dogs, cats, *etc*. Commercially important poultry may include chickens, ducks, geese, *etc*. Commercially raised fish and crustaceans such as trout and shrimp may also be included.

A content of the fermented guar meal in the feed composition according to the present disclosure may be appropriately controlled according to the kind and age of livestock, the application type, desired effects, etc., and the content may be, for example, 1% to 99% by weight, specifically 10% to 90% by weight, and more specifically 20% to 80% by weight, but is not limited thereto.

For administration, the feed composition of the present disclosure may include a mixture of one or more of organic acids such as citric acid, fumaric acid, adipic acid, lactic acid, *etc*.; phosphates such as potassium phosphate, sodium phosphate, polyphosphates, *etc*.; natural antioxidants such as polyphenol, catechin, tocopherol, vitamin C, green tea extract, chitosan, tannic acid, *etc*., in addition to the fermented guar meal. As needed, the feed composition may include other common additives such as an anti-influenza agent, a buffer, a bacteriostatic agent, *etc*. Further, the feed composition may also be formulated into injectable formulations such as an aqueous solution, a suspension, an emulsion, etc., capsules, granules, or tablets by additionally adding a diluent, a dispersant, a surfactant, a binder, or a lubricant.

Further, the feed composition of the present disclosure may include various kinds of auxiliary substances such as amino acids, inorganic salts, vitamins, antioxidants, antifungal agents, antimicrobial agents, *etc.,* main ingredients such as plant-based protein feeds such as pulverized or crushed wheat, barley, corn, etc., animal-based protein feeds such as blood meal, meat meal, fish meal, etc., animal fat and vegetable oil, as well as nutritional supplements, growth promoters, digestibility promoters, and a prophylactic agent.

When the feed composition of the present disclosure is used as a feed additive, the feed composition may be directly added or used together with other ingredients, and may be appropriately used according to a common method. The administration form of the feed composition may be prepared in an immediate release formulation or in a sustained release formulation in combination with a non-toxic pharmaceutically acceptable carrier. Such edible carriers may be corn starch, lactose, sucrose, or propylene glycol. In the case of a solid carrier, it may be administered in the form of a tablet, powder, troche, *etc*. In the case of a liquid carrier, it may be administered in the form of a syrup, a liquid suspension, an emulsion, a solution, *etc*. In addition, the administration agent may include a preservative, a lubricant, a solution promoter, a stabilizer, and may include other agents for improving inflammatory diseases and substances useful for virus prevention.

The feed composition according to the present disclosure may be mixed in an amount of about 10 g to 500 g per 1 kg, and specifically, 10 g to 100 g per 1 kg, based on the dry weight of a feed for livestock. After completely mixing, the feed composition may be provided to a mesh or may be subjected to pelletization, expansion, and extrusion processes through additional processes, but is not limited thereto.

Further, the feed composition may further include any one enzyme selected from the group consisting of galactomannanase, celluclast, and viscozyme.

Specifically, the enzyme may be included in an amount of 0.1%(w/w) to 1.0%(w/w), more specifically 0.1% to 0.5%(w/w), and much more specifically 0.2%(w/w) with respect to the weight of the fermented guar meal, considering the content of the guar gum in the fermented guar meal of the present disclosure, but is not limited thereto.

The enzyme may be an enzyme capable of reducing viscosity of the guar gum in the guar meal. When the enzyme is further included in the feed composition, 1% to 3% guar gum present in the guar meal may be degraded, thereby improving digestibility.

### Mode for Invention

Hereinafter, the present disclosure will be described in more detail with reference to Examples. However, these Examples are for illustrative purposes only, and the disclosure is not intended to be limited by these Examples.

### Example 1-1: Changes in nutritional components of guar meal according to heat treatment temperature of guar meal

A method of preparing the fermented guar meal of the present disclosure may include the step of heat-treating a water content-controlled guar meal. The bacterial count and trypsin inhibitors (TI) in the fermented guar meal according to heat treatment temperatures were measured.

In detail, 100 g of guar meal were prepared, and water was added such that the water content of the guar meal was 42%(w/w). Subsequently, the guar meal was heat-treated at 70°C, 80°C, 90°C, and 95°C for 30 minutes, respectively.

After heat treatment at different temperatures, the bacterial count and trypsin inhibitors (TI) in the guar meal were measured, and a relative ratio (%) thereof with respect to a non-heat-treated guar meal as a control group was calculated and shown in Table 1 below.

**[Table 1]**

| Changes in bacterial count and TI content according to heat treatment at different temperatures (30 minutes, based on water content of 42%) | | | | |
|---|---|---|---|---|
| Heat treatment temperature (°C) | Bacterial count | | TI content | |
| | CFU/g | Relative ratio (%) | mg/g | Relative ratio (%) |
| - | 4.0 X 10⁴ | - | 2.7 | - |
| 70 | 3.5 X 10³ | 9 | 2.1 | 79 |
| 80 | 1.7 X 10³ | 4 | 1.7 | 64 |
| 90 | 5.0 X 10² | 1 | 1.5 | 57 |
| 95 | 7.0 X 10¹ | 0 | 0.9 | 35 |

Referring to Table 1 above, as the heat treatment temperature increased, the bacterial count and the TI content in the guar meal were reduced. In particular, when heat treatment was performed at 90°C, the guar meal showed the bacterial count of 5.0 x 10² CFU/g and the TI content of 1.5 mg/g, and when heat treatment was performed at 95°C, the guar meal showed the bacterial count of 7.0 x 10¹ CFU/g and the TI content of 0.9 mg/g.

The non-heat-treated guar meal showed the bacterial count of 4.0 x 10⁴ CFU/g and the TI content of 2.7 mg/g. When heat treatment was performed at 70°C, the guar meal showed the bacterial count of 3.5 x 10³ CFU/g and the TI content of 2.1 mg/g, reaching a relative ratio of 79%. Under relatively low temperature conditions, the bacterial count and the TI content were high, indicating low availability as a feed.

In contrast, as the heat treatment temperature of the guar meal increased, the bacterial count and TI content were reduced. In particular, when the heat treatment temperature of the guar meal was 80°C to 95°C, excellent effects were observed, and when the heat treatment temperature of the guar meal was 90°C to 95°C, more excellent effects were observed. According to the results under the above heat treatment conditions, it is expected to obtain very efficient and economic effects in mass production of the fermented guar meal.

### Example 1-2: Changes in nutritional components of guar meal according to fermentation temperature and water content of guar meal

The nutritional components in the fermented guar meal according to the water content and the fermentation temperature of guar meal were measured.

In detail, 100 g of guar meal were prepared, and water was added such that the water content of the guar meal was 40%(w/w), 45%(w/w), and 50%(w/w), respectively. Subsequently, the guar meals were heat-treated at 90°C for 30 minutes, and then cooled to 40°C, respectively.

A culture of *Bacillus amyloliquefaciens* K2G was seeded in each of the guar meals having different water contents in an amount of 10 % by weight based on the weight of the guar meal, and mixed well such that the water content was 40%(w/w), 45%(w/w), and 50%(w/w), respectively. Then, the guar meals were fermented at 35°C, 40°C, 45°C, and 50°C for 16 hours under the conditions where the temperature and humidity were constantly maintained. A guar meal raw material group which did not undergo the water content-controlling step, the heat treatment step, and the fermentation step according to the present disclosure was used as a control group.

Protein contents in the guar meals according to the water content and the fermentation temperature were measured, and an increase ratio and a relative ratio of the protein content with respect to the guar meal raw material group were calculated and shown in Table 2 below.

**[Table 2]**

| Changes in nutritional components according to fermentation temperature and water content (*Bacillus amyloliquefaciens* K2G, fermentation time of 16 hours) | | | | |
|---|---|---|---|---|
| Fermentation temperature (°C) | Water content (%) | Protein content (%, based on ds) | | |
| | | | Increase ratio (%) | Relative ratio (%) |
| - | - | 57.2 | - | - |
| | 40 | 58.9 | 1.7 | 103 |
| 35°C | 45 | 61.7 | 4.5 | 108 |
| | 50 | 62.2 | 5.0 | 109 |
| | 40 | 61.0 | 3.8 | 107 |
| 40°C | 45 | 63.2 | 6.0 | 110 |
| | 50 | 63.5 | 6.3 | 111 |
| | 40 | 60.0 | 2.8 | 105 |
| 45°C | 45 | 62.9 | 5.7 | 110 |
| | 50 | 62.5 | 5.3 | 109 |
| | 40 | 58.7 | 1.5 | 103 |
| 50°C | 45 | 60.0 | 2.8 | 105 |
| | 50 | 60.6 | 3.4 | 106 |

Referring to Table 2, under conditions of the fermentation temperature of 40°C and the water content of 50%, the highest protein content of 63.5% and the highest increase ratio of 110% were observed. In other words, it was confirmed that when the guar meals were fermented at 35°C to 50°C, or 40°C to 45°C, they had the high protein content. It was also confirmed that when the guar meals were fermented after being treated to have the water content of 40% to 50% or 45% to 50%, they had the high protein contents.

### Example 1-3: Changes in nutritional components of fermented guar meal according to strains

100 g of guar meal were prepared, and water was added such that the water content of the guar meal was 40%(w/w) to 45%(w/w). Subsequently, the guar meal was heat-treated at 80°C to 95°C for 30 minutes, and then cooled to 40°C. Each culture of one kind of *Bacillus subtilis* (*Bacillus subtilis* ATCC 21770) and four kinds of *Bacillus amyloliquefaciens* (*Bacillus amyloliquefaciens* K2G, ATCC 23842, ATCC 23843, and ATCC 23845) was seeded in the cooled guar meal in an amount of 10% by weight based on the weight of the guar meal, and mixed well such that the final fermentation water content was 45%(w/w) to 50%(w/w). Then, the guar meals were fermented at 37°C to 45°C for 14 hours to 16 hours under the conditions where the temperature and humidity were constantly maintained. A guar meal raw material group which did not undergo the water content-controlling step, the heat treatment step, and the fermentation step according to the present disclosure was used as a control group.

Protein contents, live bacterial count, and pH of the fermented guar meals were measured, and an increase ratio and a relative ratio of the protein content with respect to the guar meal raw material group were calculated and shown in Table 3 below.

**[Table 3]**

| Changes in nutritional components according to strain treatment (based on fermentation time of 16 hours) | | | | | |
|---|---|---|---|---|---|
| Raw material and strain | Protein content (%, based on ds) | | | Live bacterial count (CFU/g) | pH |
| | | Increase ratio (%) | Relative ratio (%) | | |
| Guar meal | 57.2 | - | - | - | 6.6 |
| *Bacillus subtilis* ATCC 21770 | 62.3 | 5.1 | 88 | 8.9 X 10⁹ | 7.1 |
| *Bacillus amyloliquefaciens* ATCC 23842 | 62.3 | 5.1 | 88 | 1.2 X 10¹⁰ | 7.4 |
| *Bacillus amyloliquefaciens* ATCC 23845 | 62.5 | 5.3 | 91 | 1.2 X 10¹⁰ | 7.5 |
| *Bacillus amyloliquefaciens* ATCC 23843 | 62.9 | 5.7 | 98 | 1.2 X 10¹⁰ | 7.5 |
| *Bacillus amyloliquefaciens* K2G | 63.0 | 5.8 | 100 | 1.2 X 10¹⁰ | 7.6 |

Referring to Table 3, the guar meals fermented by seeding the *Bacillus* strains were found to include 62% or more of crude proteins, which is 5% or higher than that of the guar meal of the control group in which the *Bacillus* strain was not seeded. Therefore, when the fermented guar meal is added to a feed, a feed blending ratio may be reduced. Further, the live bacterial count in the fermented guar meal reached 10⁷ CFU/g to 10⁸ CFU/g. Therefore, when the fermented guar meal is added to a feed, probiotics effects may be expected.

### Example 2: Degradation of guar gum in guar meal and change of viscosity thereof

1% to 3% guar gum is present in guar meal, and viscosity of guar gum is known to slow digestion in the digestive organs, thereby inhibiting digestibility. Therefore, an enzyme capable of reducing viscosity of the guar gum in the guar meal may be added to the fermented guar meal of the present disclosure.

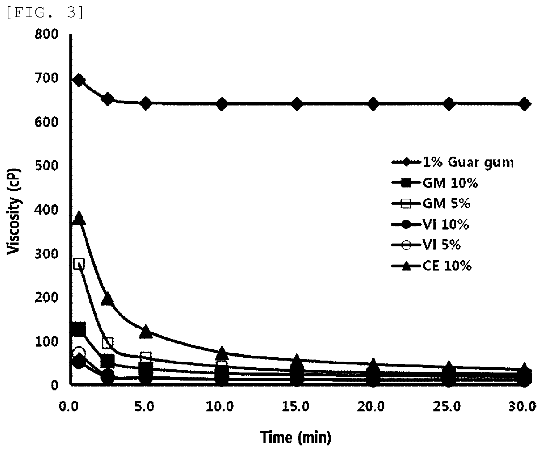

Degradation of guar gum (galactomannan) or reduction of viscosity thereof according to the kind and concentration of enzymes were examined. In detail, guar gum was dissolved in sterile water to prepare a 1% guar gum dilution. Each enzyme of galactomannanase (hereinafter, referred to as GM), celluclast (hereinafter, referred to as CE), and viscozyme (hereinafter, referred to as VI) was added at different concentrations to the guar gum dilution, and changes in the viscosity of the guar gum solution according to degradation of the guar gum were examined (FIG. 3).

As a result, it was confirmed that all of GM, CE, and VI reduced the viscosity of the guar gum, and in particular, GM and VI showed excellent effects of reducing the viscosity of the guar gum. Therefore, when the enzyme is further included in a feed composition including the fermented guar meal prepared by the preparation method of the present disclosure, it is possible to provide a feed composition capable of improving digestibility and an absorption rate of a feed.

### Example 3: Changes in sugar content in guar meal according to fermentation with Bacillus

A guar meal includes 4% to 6% oligosaccharides. Among these oligosaccharides, raffinose and stachyose which are indigestible oligosaccharides, *i.e.,* galacto-oligosaccharides (hereinafter, referred to as GOS) occupy 2% to 3%. The indigestible oligosaccharides may be used as a nutritional source of harmful intestinal microorganisms or may cause diarrhea. Thus, it is necessary to reduce the content thereof in the guar meal.

100 g of guar meal were prepared, and water was added such that the water content of the guar meal was 40%(w/w) to 45%(w/w). Subsequently, the guar meal was heat-treated at 80°C to 95°C for 30 minutes, and then cooled to 40°C. Each culture of one kind of *Bacillus subtilis* (*Bacillus subtilis* ATCC 21770) and four kinds of *Bacillus amyloliquefaciens* (*Bacillus amyloliquefaciens* K2G, ATCC 23842, ATCC 23843, and ATCC 23845), *Saccharomyces cerevisiae,* and *Lactobacillus acidophilus,* and a mixed culture of *Saccharomyces* and *Lactobacillus acidophilus* was seeded in an amount of 10% based on the weight of the guar meal, respectively. Then, the guar meals to which each strain was seeded were mixed well, and then fermented at 37°C to 45°C for 14 hours to 16 hours under the conditions where the temperature and humidity were constantly maintained. However, the guar meals to which the culture of *Saccharomyces cerevisiae,* the culture of *Lactobacillus acidophilus,* or the mixed culture of *Saccharomyces* and *Lactobacillus acidophilus* was seeded were fermented under anaerobic conditions.

Water extraction was performed for each of the fermented guar meals fermented for the same period of time, and filtered through a 0.22 µm filter. The contents of indigestible oligosaccharides were measured using a Carbo PA-1 (a column temperature of 30°C). A guar meal raw material group which did not undergo the water content-controlling step, the heat treatment step, and the fermentation step according to the present disclosure was used as a control group. The contents of indigestible oligosaccharides in the fermented guar meals were measured, and a relative ratio thereof with respect to the guar meal raw material group was calculated and shown in Table 4 below.

**[Table 4]**

| Contents of residual indigestible oligosaccharides according to strain treatment | | | | |
|---|---|---|---|---|
| Raw material and strain | Relative ratio (%) | Indigestible oligosaccharides | | |
| | | Raffinose (%) | Stachyose (%) | Total (%) |
| Guar meal | 100 | 1.71 | 0.51 | 2.22 |
| *Bacillus subtilis* ATCC 21770 | 39.6 | 0.20 | 0.68 | 0.88 |
| *Bacillus amyloliquefaciens* ATCC 23842 | 30.2 | 0.02 | 0.65 | 0.67 |
| *Bacillus amyloliquefaciens* ATCC 23845 | 19.4 | 0.02 | 0.41 | 0.43 |
| *Bacillus amyloliquefaciens* ATCC 23843 | 15.3 | ND¹⁾ | 0.34 | 0.34 |
| *Bacillus amyloliquefaciens* K2G | 10.4 | ND | 0.23 | 0.23 |
| *Saccharomyces* | 71.2 | 1.00 | 0.58 | 1. 58 |
| *cerevisiae* | | | | |
| *Lactobacillus acidophilus* | 89.2 | 1.46 | 0.52 | 1. 98 |
| *Saccharomyces cerevisiae + Lactobacillus acidophilus* | 71.2 | 1.11 | 0.47 | 1.58 |

| | | | | |
|---|---|---|---|---|
| ¹⁾ND: not detected | | | | |

Referring to Table 4, the contents of indigestible oligosaccharides in the fermented guar meal to which the *Bacillus* strain was seeded showed 80% or more reduction on average, as compared with the control group. In particular, the guar meal fermented with *Bacillus amyloliquefaciens* K2G showed the lowest contents of indigestible oligosaccharides. In contrast, from the guar meal fermented with the culture of *Saccharomyces cerevisiae,* the culture of *Lactobacillus acidophilus,* or the mixed culture thereof under anaerobic conditions, GOS was removed to 20% to 30% on average. Therefore, since the fermented guar meals of the present disclosure have the low contents of indigestible oligosaccharides, they may be used as feeds to improve digestibility of livestock.

### Example 4: Changes in TI content and in vitro digestibility according to fermentation with Bacillus

The content of trypsin inhibitor (hereinafter, referred to as TI) in the fermented guar meal prepared according to the present disclosure and *in vitro* digestibility were measured.

In detail, 100 g of guar meal were prepared, and water was added such that the water content of the guar meal was 40% (w/w) to 45% (w/w). Subsequently, the guar meal was heat-treated at 90°C for 30 minutes, and then cooled to 40°C. Each culture of one kind of *Bacillus subtilis* (*Bacillus subtilis* ATCC 21770), four kinds of *Bacillus amyloliquefaciens* (*Bacillus amyloliquefaciens* K2G, ATCC 23842, ATCC 23843, and ATCC 23845), *Saccharomyces cerevisiae,* and *Lactobacillus acidophilus,* and a mixed culture of *Saccharomyces* and *Lactobacillus acidophilus* was seeded in the guar meal in an amount of 10 % by weight based on the weight of the guar meal. The guar meals to which each strain was seeded were mixed well, respectively and fermented at 37°C to 45°C for 16 hours under the conditions where the temperature and humidity were constantly maintained. However, the guar meals to which the culture of *Saccharomyces cerevisiae,* the culture of *Lactobacillus acidophilus,* or the mixed culture of *Saccharomyces* and *Lactobacillus acidophilus* was seeded were fermented under anaerobic conditions. A guar meal raw material group which did not undergo the water content-controlling step, the heat treatment step, and the fermentation step according to the present disclosure was used as a control group.

TI contents in the guar meals fermented by the method and in the control group were measured in accordance with AOAC Ba 12-75 (American Oil Chemists' Society), and *in vitro* digestibility was measured in accordance with Biosen et al (1997) 2 step. The measured TI content (mg/g) is shown in Table 5 below, and *in vitro* digestibility is shown in Table 6 below.

**[Table 5]**

| Contents of anti-nutritional factor TI according to strain treatment | | |
|---|---|---|
| Raw material and strain | TI content (mg/g) | |
| | | Relative ratio (%) |
| Guar meal | 2.5 | 100 |
| *Bacillus subtilis* ATCC 21770 | 1.1 | 45 |
| *Bacillus amyloliquefaciens* ATCC 23842 | 1.1 | 45 |
| *Bacillus amyloliquefaciens* ATCC 23845 | 1.1 | 45 |
| *Bacillus amyloliquefaciens* ATCC 23843 | 0.9 | 36 |
| *Bacillus amyloliquefaciens* K2G | 0.9 | 36 |
| *Saccharomyces cerevisiae* | 1.6 | 65 |
| *Lactobacillus acidophilus* | 1.9 | 77 |
| *Saccharomyces cerevisiae + Lactobacillus* | 1.6 | 65 |
| *acidophilus* | | |

Referring to Table 5, the guar meals fermented with the *Bacillus* strains according to the present disclosure had the TI content of 1.0 mg/g on average, indicating 60% reduction on average, as compared with the guar meal raw material. In contrast, the effect of reducing the TI content by fermentation was not significant in the guar meals fermented with the culture of *Saccharomyces cerevisiae,* the culture of *Lactobacillus acidophilus,* and a mixed culture thereof under anaerobic conditions, as compared with the guar meal fermented with the culture of the *Bacillus* strain. Therefore, the method of preparing the fermented guar meal of the present disclosure, the method including the step of fermenting the guar meal with the culture of the *Bacillus* strain, may reduce the TI content in the guar meal. Thus, when the fermented guar meal of the present disclosure may be used as a feed, the protein digestibility of livestock may be improved.

**[Table 6]**

| Changes in *in vitro* digestibility (%) according to strain treatment | | | |
|---|---|---|---|
| Raw material and strain | *In vitro* digestibility (%) | | |
| | 1 step (pepsin) | 2 steps (pepsin + pancreatin) | Relative ration (%) |
| Guar meal | 81.0 | 87 | - |
| *Bacillus subtilis* ATCC 21770 | 85.0 | 91 | 105 |
| *Bacillus amyloliquefaciens* ATCC 23843 | 84.0 | 90 | 103 |
| *Bacillus amyloliquefaciens* K2G | 89.0 | 95 | 109 |
| *Saccharomyces cerevisiae* | 81.0 | 87 | 100 |
| *Lactobacillus acidophilus* | 82.0 | 86 | 99 |

Referring to Table 6, the guar meals fermented with the culture of the *Bacillus* strain showed high *in vitro* digestibility, as compared with the guar meal fermented with the culture of *Lactobacillus acidophilus,* the guar meal fermented with the mixed culture of *Saccharomyces* and *Lactobacillus acidophilus,* and the control guar meal, and this may be attributed to low molecularization of guar meal proteins in the step of fermenting the guar meal with the culture of the *Bacillus* strain. However, *Saccharomyces cerevisiae* and *Lactobacillus acidophilus* appears to be unable to cause low molecularization of guar meal proteins, unlike the *Bacillus* strains. Therefore, when the fermented guar meal prepared by the method of preparing the fermented guar meal of the present disclosure is used as a feed, the protein digestibility of livestock may be improved.

### Example 5: Changes in hydrolysis of guar meal protein according to fermentation with Bacillus

A guar meal has a protein content similar to or higher than that of a soybean meal. However, since the guar meal includes a large amount of anti-nutritional factors and high-molecular weight proteins with low digestibility, it has a disadvantage that its use for young livestock is difficult due to low digestibility. The proteins with low digestibility may be improved by converting the proteins into hydrolyzed peptides or degrading the proteins into low molecular weight proteins that are easy to digest.

Therefore, in order to examine improvement of digestibility of the fermented guar meal prepared according to the method of the present disclosure, the degree of hydrolysis or the molecular weight of the proteins of the fermented guar meal were measured.

In detail, 100 g of guar meal were prepared, and water was added such that the water content of the guar meal was 40 %(w/w) to 45 %(w/w). Subsequently, the guar meal was heat-treated at 90°C for 30 minutes, and then cooled to 40°C. Each culture of *Bacillus amyloliquefaciens* K2G, *Saccharomyces cerevisiae,* and *Lactobacillus acidophilus,* or a mixed culture of *Saccharomyces* and *Lactobacillus acidophilus* was seeded in the guar meal in an amount of 10 % by weight based on the weight of the guar meal. The guar meals to which each strain was seeded were mixed well, respectively and fermented at 37°C to 45°C for 16 hours under the conditions where the temperature and humidity were constantly maintained. However, the guar meals to which the culture of *Saccharomyces cerevisiae,* the culture of *Lactobacillus acidophilus,* or the mixed culture of *Saccharomyces* and *Lactobacillus acidophilus* was seeded were fermented under anaerobic conditions.

5.0 mL of 8 M urea solution was added to and mixed with 0.1 g of a dry sample of each of the obtained fermented guar meals, and centrifuged at 25°C and 13000 rpm for 5 minutes to separate each supernatant. Each of the separated supernatants was stained and denatured with a staining buffer, and 10 µl thereof was subjected to SDS-PAGE to examine the protein mobility according to a molecular weight.

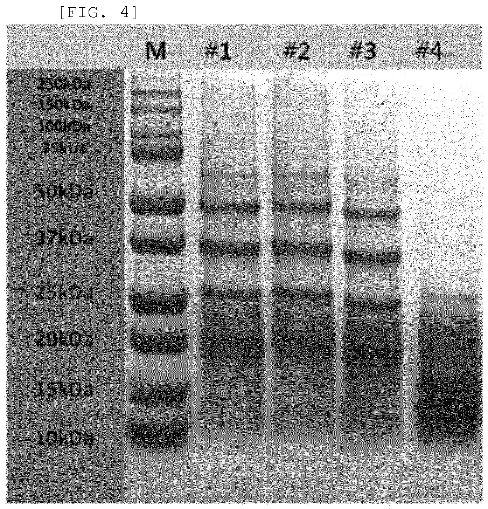

FIG. 4 is a graph showing degree of hydrolysis of guar meal proteins according to the kind of strains. From the left, line 1 represents an extraction supernatant of a guar meal raw material group which did not undergo the water content-controlling step, the heat treatment step, and the fermentation step according to the present disclosure, line 2 represents an extraction supernatant of the guar meal fermented with *Saccharomyces cerevisiae,* line 3 represents an extraction supernatant of the guar meal fermented with *Lactobacillus acidophilus,* and line 4 represents an extraction supernatant of the guar meal fermented with *Bacillus amyloliquefaciens* K2G.

As a result, the fermented guar meal prepared using *Bacillus amyloliquefaciens* K2G showed a remarkable reduction in the molecular weight, as compared with the guar meal raw material group or the fermented guar meals prepared using other strains. The Bacillus strains hydrolyzed the high-molecular-weight proteins of the guar meal into peptides having a small size, and therefore, the fermented guar meal of the present disclosure may be used as a protein source for young livestock.

### Example 6: Changes in nutritional components of mixture of guar meal and soybean meal according to fermentation with Bacillus

A guar meal (GM) and a soybean meal (SBM) were blended at a ratio of 1:9, 5:5, and 9:1 to prepare mixtures, and water was added such that the water content of each mixture was 40 % (w/w) to 45 %(w/w). Subsequently, each mixture was mixed well, and then heat-treated at 90°C to 95°C for 25 minutes to 30 minutes. After heat treatment, each mixture was cooled. Each culture of two kinds of *Bacillus amyloliquefaciens* (*Bacillus amyloliquefaciens* K2G and ATCC 23843) was seeded in the cooled mixture of guar meal/soybean meal in an amount of 5% to 20 % by weight, and then mixed well, respectively and fermented at 37°C to 45°C for 14 hours to 16 hours under the conditions where the temperature and humidity were constantly maintained. The nutritional components of the fermented guar meal/soybean meal mixture are shown in Table 7 below.

**[Table 7]**

| Changes in nutritional components after fermentation according to blending ratio of guar meal and soybean meal | | | | | | | |
|---|---|---|---|---|---|---|---|
| Raw material (ratio) and strain | Protein content (%, based on ds) | | | Live bacterial count (CFU/g) | pH | TI (mg/g) | GOS (%, R.+S.*) |
| | | Increase ratio (%) | Relative ratio (3) | | | | |
| *Raw materials* | | | | | | | |
| GM1:SBM9 | 54.6 | - | - | - | 6.6 | 4.1 | 5.2 |
| GM5:SBM5 | 55.8 | - | - | - | 6.2 | 3.5 | 3.9 |
| GM9:SBM1 | 56.9 | - | - | - | 6.5 | 2.9 | 2.5 |

| *Bacillus amyloliquefaciens* ATCC 23843 | | | | | | | |
|---|---|---|---|---|---|---|---|
| GM1:SBM9 | 60.7 | 6.1 | 93.8 | 1.1 X 10¹⁰ | 7.2 | 0.6 | 0.4 |
| GM5:SBM5 | 61.5 | 5.7 | 87.7 | 9.7 X 10⁹ | 7.2 | 0.5 | 0.6 |
| GM9:SBM1 | 62.3 | 5.4 | 83.1 | 1.1 X 10¹⁰ | 6.9 | 0.6 | 0.5 |

| *Bacillus amyloliquefaciens* K2G | | | | | | | |
|---|---|---|---|---|---|---|---|
| GM1:SBM9 | 61.1 | 6.5 | 100.0 | 1.4 X 10¹⁰ | 7.4 | 0.5 | 0.3 |
| GM5:SBM5 | 62.2 | 6.4 | 98.5 | 1.2 X 10¹⁰ | 7.2 | 0.5 | 0.4 |
| GM9:SBM1 | 62.8 | 5.9 | 90.8 | 1.1 X 10¹⁰ | 7.2 | 0.8 | 0.4 |

| | | | | | | | |
|---|---|---|---|---|---|---|---|
| *The contents of Raffinose and Stachyose | | | | | | | |

As a result, both of the guar meals fermented with *Bacillus amyloliquefaciens* (K2G or ATCC23843) showed 5.4% to 6.5% increase in the content of crude protein, as compared with that of the raw material guar meal, and both of the *Bacillus* strains were able to grow with the live bacterial count of 10 log. Further, the contents of the anti-nutritional factor TI and GOS were reduced, as compared with that of the raw material. All of them showed pH increase, which is attributed to ammonia produced during a metabolic process according to protease activity of *Bacillus.*

Therefore, the fermented guar meal/soybean meal mixture according to the present disclosure may be very suitable for *Bacillus* fermentation, and through fermentation, the content of crude protein was increased and the contents of the anti-nutritional factor TI and GOS were reduced, thereby greatly improving digestibility.

### Example 7: Evaluation of fermented guar meal by broiler breeding test

To evaluate the effects of the fermented guar meals, a broiler breeding test was performed. To perform the breeding test, broilers were acclimated for 4 days, and fed for total 21 days (3 weeks) with a soybean meal or a feed in which 3%, 5%, or 7% of soybean meal was replaced by the fermented guar meal, and the broiler's body weight and feed intake were examined. The test was designed using total 80 broilers of 4 treatments X 4 repeats X 5 birds.

In detail, 100 g of guar meal were prepared, and water was added such that the water content of the guar meal was 40 %(w/w) to 45 %(w/w). Subsequently, the guar meal was heat-treated at 90°C for 30 minutes, and then cooled to 40°C. The culture of *Bacillus amyloliquefaciens* K2G was seeded in the guar meal in an amount of 10 % by weight based on the weight of the guar meal. The guar meal to which the strain was seeded was mixed well, and fermented at 37°C to 45°C for 14 hours to 16 hours under the conditions where the temperature and humidity were constantly maintained, followed by drying. Broilers were fed with each test sample prepared by blending the soybean meal with the fermented guar meal according to the replacement level. The results of the broiler breeding test according to feeding of the fermented guar meal are shown in Table 8 below.

**[Table 8]**

| Results of broiler breeding test of fermented guar meal | | | | |
|---|---|---|---|---|
| Treatment group | T1 | T2 | T3 | T4 |
| | Basal diet | Fermented guar meal | | |
| | (CP 22%) | | | |
| Replacement level, % | - | 3% | 5% | 7% |
| BW, g/bird | | | | |
| 5-day-old | 71.7 | 72 | 72.1 | 72.1 |
| 21-day-old | 458.9 | 486 | 485.9 | 452.7 |
| 5 to 21-day-old | | | | |
| BWG, g/bird | 387 | 414 | 414 | 381 |
| FI, g/bird | 629 | 680 | 699 | 673 |
| FCR | 1.64 | 1.65 | 1. 69 | 1.78 |

As a result, when the fermented guar meal prepared using *Bacillus amyloliquefaciens* K2G was replaced by 5%, the broiler's body weight and feed intake (FI) were increased, as compared with those fed with only the soybean meal. That is, these results indicate that there is no problem in preference for the fermented guar meal, and a strong grass smell which is the raw guar meal's own smell was reduced, and its feed conversion ratio (FCR) was also similar to that of the soybean meal. Therefore, the fermented guar meal prepared according to the present disclosure may be widely used in the future as a protein source to replace the soybean meal.

## Claims

1. A method of preparing a fermented guar meal, the method comprising the following steps of:
(1) pretreating a guar meal;
(2) seeding a *Bacillus* strain in an amount of 5% by weight to 20% by weight based on the weight of the guar meal in the pretreated guar meal;
(3) solid-state fermenting the strain seeded in the guar meal to obtain the fermented guar meal; and
(4) drying and pulverizing the fermented guar meal;
wherein the pretreatment step of the step (1) includes the steps of (1-1) adding water to the guar meal to control a water content thereof; (1-2) heat-treating the water content-controlled guar meal; and (1-3) cooling the heat-treated guar meal,
wherein the water content-controlled guar meal of step (1-1) has a water content of 30% to 60%(w/w),
wherein the heat treatment of the step (1-2) is performed at 85°C to 100°C for 20 minutes to 35 minutes,
wherein the cooling of the step (1-3) is performed at a temperature of 20°C to 60°C,
and wherein the Bacillus strain is *Bacillus amyloliquefaciens.*

2. The method of claim 1, wherein the water content of the guar meal after the step (1-1) is 40% to 45%.

3. The method of claim 1, wherein the heat treatment of the step (1-2) is performed at 90°C to 95°C.

4. The method of claim 1, wherein the cooling of the step (1-3) is to perform cooling to a temperature at which solid-state fermentation is possible.

5. The method of claim 1, wherein the solid-state fermenting of the step (3) is performed at a temperature of 30°C to 50°C for 15 hours to 25 hours.

6. The method of claim 1, wherein the fermented guar meal includes trypsin inhibitors (TI) of 30% to 50%, based on a content of trypsin inhibitors in a guar meal which does not undergo the steps (1) to (3), or the fermented guar meal includes indigestible oligosaccharides of 10% to 40%, based on a content of indigestible oligosaccharides in the guar meal which does not undergo the steps (1) to (3).

7. A fermented guar meal prepared by the method of any one of claims 1 to 6.

8. The fermented guar meal of claim 7, wherein the fermented guar meal includes trypsin inhibitors (TI) of 30% to 50%, based on a content of trypsin inhibitors in a guar meal which does not undergo the steps (1) to (3) of claim 1, or the fermented guar meal includes indigestible oligosaccharides of 10% to 40%, based on a content of indigestible oligosaccharides in the guar meal which does not undergo the steps (1) to (3) of claim 1.

9. A feed composition comprising a fermented guar meal prepared by the method of any one of claims 1 to 6.

10. The feed composition of claim 9, further comprising any one enzyme selected from the group consisting of galactomannanase, celluclast, and viscozyme.

11. The feed composition of claim 10, wherein the enzyme is included in an amount of 0.1 %(w/w) to 1.0 %(w/w), based on the weight of the fermented guar meal.

## Patentansprüche

1. Verfahren zur Herstellung eines fermentierten Guarkernmehls, wobei das Verfahren die folgenden Schritte umfasst:
(1) Vorbehandeln eines Guarkernmehls;
(2) Aussäen eines Bacillus-Stammes in einer Menge von 5 Gew.-% bis 20 Gew.-%, bezogen auf das Gewicht des Guarkernmehls, in das vorbehandelte Guarkernmehl;
(3) Feststoff-Fermentation des in das Guarkernmehl gesäten Stammes, um das fermentierte Guarkernmehl zu erhalten; und
(4) Trocknen und Pulverisieren des fermentierten Guarkernmehls;
wobei der Vorbehandlungsschritt des Schritts (1) die folgenden Schritte umfasst: (1-1) Zugabe von Wasser zu dem Guarkernmehl, um dessen Wassergehalt zu kontrollieren; (1-2) Wärmebehandlung des wassergehaltskontrollierten Guarkernmehls; und (1-3) Abkühlen des wärmebehandelten Guarkernmehls,
wobei das wassergehaltskontrollierte Guarkernmehl aus Schritt (1-1) einen Wassergehalt von 30% bis 60% (w/w) aufweist,
wobei die Wärmebehandlung aus Schritt (1-2) bei 85°C bis 100°C für 20 Minuten bis 35 Minuten durchgeführt wird,
wobei das Abkühlen aus Schritt (1-3) bei einer Temperatur von 20°C bis 60°C durchgeführt wird,
und wobei es sich bei dem Bacillus-Stamm um Bacillus amyloliquefaciens handelt.

2. Verfahren nach Anspruch 1, wobei der Wassergehalt des Guarkernmehls nach Schritt (1-1) 40 % bis 45 % beträgt.

3. Verfahren nach Anspruch 1, wobei die Wärmebehandlung aus Schritt (1-2) bei 90°C bis 95°C durchgeführt wird.

4. Verfahren nach Anspruch 1, wobei die Abkühlung aus Schritt (1-3) dazu dient, eine Abkühlung auf eine Temperatur durchzuführen, bei der eine Feststoff-Fermentation möglich ist.

5. Verfahren nach Anspruch 1, wobei die Feststoff-Fermentation aus Schritt (3) bei einer Temperatur von 30°C bis 50°C für 15 Stunden bis 25 Stunden durchgeführt wird.

6. Verfahren nach Anspruch 1, wobei das fermentierte Guarkernmehl 30 % bis 50 % Trypsininhibitoren (TI) enthält, bezogen auf einen Gehalt an Trypsininhibitoren in einem Guarkernmehl, das nicht den Schritten (1) bis (3) unterzogen wird, oder das fermentierte Guarkernmehl unverdauliche Oligosaccharide von 10 % bis 40 % enthält, bezogen auf einen Gehalt an unverdaulichen Oligosacchariden in dem Guarkernmehl, das nicht den Schritten (1) bis (3) unterzogen wird.

7. Fermentiertes Guarkernmehl, hergestellt nach dem Verfahren gemäß einem der Ansprüche 1 bis 6.

8. Fermentiertes Guarkernmehl nach Anspruch 7, wobei das fermentierte Guarkernmehl 30 % bis 50 % Trypsininhibitoren (TI) enthält, bezogen auf den Gehalt an Trypsininhibitoren in einem Guarkernmehl, das nicht den Schritten (1) bis (3) nach Anspruch 1 unterzogen wird, oder das fermentierte Guarkernmehl unverdauliche Oligosaccharide von 10 % bis 40 % enthält, bezogen auf einen Gehalt an unverdaulichen Oligosacchariden in dem Guarkernmehl, das nicht den Schritten (1) bis (3) nach Anspruch 1 unterzogen wird.

9. Futtermittelzusammensetzung, umfassend ein fermentiertes Guarkernmehl, das nach dem Verfahren gemäß einem der Ansprüche 1 bis 6 hergestellt wurde.

10. Futtermittelzusammensetzung nach Anspruch 9, die ferner ein beliebiges Enzym enthält, das aus der Gruppe ausgewählt ist, die aus Galactomannanase, Celluclast und Viskozym besteht.

11. Futtermittelzusammensetzung nach Anspruch 10, wobei das Enzym in einer Menge von 0,1 % (w/w) bis 1,0 % (w/w), bezogen auf das Gewicht des fermentierten Guarkernmehls, enthalten ist.

## Revendications

1. Procédé de préparation d'une farine de guar fermentée, le procédé comprenant les étapes suivantes:
(1) prétraitement d'une farine de guar;
(2) ensemencement d'une souche de Bacillus en une quantité de 5% en poids à 20% en poids par rapport au poids de la farine de guar dans la farine de guar prétraitée;
(3) fermentation à l'état solide de la souche ensemencée dans la farine de guar pour obtenir la farine de guar fermentée; et
(4) séchage et pulvérisation de la farine de guar fermentée;
dans lequel l'étape de prétraitement de l'étape (1) comprend les étapes de (1-1) addition d'eau à la farine de guar pour contrôler sa teneur en eau; (1-2) traitement thermique de la farine de guar à teneur en eau contrôlée; et (1-3) refroidissement de la farine de guar traitée thermiquement,
dans lequel la farine de guar à teneur en eau contrôlée de l'étape (1-1) a une teneur en eau de 30% à 60% (p/p),
dans lequel le traitement thermique de l'étape (1-2) est effectué à 85°C à 100°C pendant 20 minutes à 35 minutes,
dans lequel le refroidissement de l'étape (1-3) est effectué à une température de 20°C à 60°C,
et dans lequel la souche de Bacillus est Bacillus amyloliquefaciens.

2. Le procédé selon la revendication 1, dans lequel la teneur en eau de la farine de guar après l'étape (1-1) est de 40% à 45%.

3. Le procédé selon la revendication 1, dans lequel le traitement thermique de l'étape (1-2) est effectué à 90°C à 95°C.

4. Le procédé selon la revendication 1, dans lequel le refroidissement de l'étape (1-3) consiste à effectuer un refroidissement jusqu'à une température à laquelle la fermentation à l'état solide est possible.

5. Le procédé selon la revendication 1, dans lequel la fermentation à l'état solide de l'étape (3) est effectuée à une température de 30°C à 50°C pendant 15 heures à 25 heures.

6. Le procédé selon la revendication 1, dans lequel la farine de guar fermentée comprend des inhibiteurs de trypsine (TI) de 30% à 50%, par rapport à une teneur en inhibiteurs de trypsine dans une farine de guar qui ne subit pas les étapes (1) à (3), ou la farine de guar fermentée comprend des oligosaccharides indigestibles de 10% à 40%, par rapport à une teneur en oligosaccharides indigestibles dans la farine de guar qui ne subit pas les étapes (1) à (3).

7. Farine de guar fermentée préparée par le procédé selon l'une quelconque des revendications 1 à 6.

8. La farine de guar fermentée selon la revendication 7, dans laquelle la farine de guar fermentée comprend des inhibiteurs de trypsine (TI) de 30% à 50%, par rapport à une teneur en inhibiteurs de trypsine dans une farine de guar qui ne subit pas les étapes (1) à (3) de la revendication 1, ou la farine de guar fermentée comprend des oligosaccharides indigestibles de 10% à 40%, par rapport à une teneur en oligosaccharides indigestibles dans la farine de guar qui ne subit pas les étapes (1) à (3) de la revendication 1.

9. Composition d'aliment comprenant une farine de guar fermentée préparée par le procédé selon l'une des revendications 1 à 6.

10. La composition d'aliment selon la revendication 9, comprenant en outre une enzyme quelconque choisie dans le groupe constitué de la galactomannannase, du celluclast et de la viscozyme.

11. La composition d'aliment selon la revendication 10, dans laquelle l'enzyme est incluse en une quantité de 0,1% (p/p) à 1,0% (p/p), par rapport au poids de la farine de guar fermentée.
